# EUROPEAN PATENT SPECIFICATION

(11) **EP 2 512 565 B1**
(45) Date of publication and mention of the grant of the patent: **14.06.2017**
(21) Application number: 10816411.2
(22) Date of filing: 09.12.2010
(51) Int. Cl.: A61M 11/00

(54) **NEBULIZER WITH A REDUCED NUMBER OF COMPONENTS**
ZERSTÄUBER MIT REDUZIERTER ANZAHL AN BESTANDTEILEN
NÉBULISEUR COMPORTANT UN NOMBRE RÉDUIT DE COMPOSANTS

(30) Priority: 15.12.2009 IT MI20090411 U
(43) Date of publication of application: 24.10.2012
(73) Proprietor: Artsana S.p.A., 22070 Grandate (CO) (IT)
(72) Inventor: MAURI, Elena, I-22100 Como (IT); POLLACI, Daniele, I-22070 Grandate (CO) (IT)
(74) Representative: Bonvicini, Davide
(86) International application number: PCT/IB2010/003172
(87) International publication number: WO 2011/073756

(56) References cited:
- WO-A1-01/28614
- US-A1- 2003 197 068
- US-A1- 2004 031 485

## Description

The present invention relates to a nebulizer in accordance with the introduction to the main claim.

Nebulizers for medical use enabling medical products to be inhaled, of the type comprising a body with a first cup-shaped part or portion for containing the product to be dispensed, have been long known. Extending upwards from a bottom part of this portion there is a hollow tubular body, the cavity of which is arranged to cooperate with a generator generating a forced flow of a fluid (generally air). This cavity opens at a free end of the tubular body. A tube is coaxially fitted onto this latter in such a manner as to form with said body a space through which the product in the reservoir is able to rise towards the free end of the body. This rising movement, generated by the venturi effect of the fluid rising through said cavity, causes the product to emerge, nebulized into minute droplets, from apertures present in a part of the tube positioned transversely above the end opening of the tubular body.

Above this opening a deflector element is positioned to cause the fluid and product flow, emerging from the apertures, to move towards a dispenser in a second part of the nebulizer body. The heavier product droplets do not move towards the dispenser but fall into the reservoir after striking the deflector.

US 2003/0197068 refers to a nebulizer as above described. To this prior document the introductory part of the main claim refers.

As also described in the prior art the tube positioned on the tubular body is usually an independent element, separate from the other nebulizer components. As the dimensions of that component are generally very small, after cleaning the nebulizer (done by disassembling it into its various components) it can frequently be lost or forgotten during reassembly. Moreover this tube can be easily broken if squashed involuntarily by not being seen.

Finally the production of such a separate tube has a cost which has to be considered in the nebulizer sale price.

An object of the present invention is to provide a nebulizer which is of economical production, is highly practical in use, and enables optimal product dispensing.

This and other objects which will be apparent to the expert of the art are attained by a nebulizer in accordance with the accompanying claims.

The present invention will be better understood from the accompanying drawing, which is provided by way of non-limiting example, and in which:

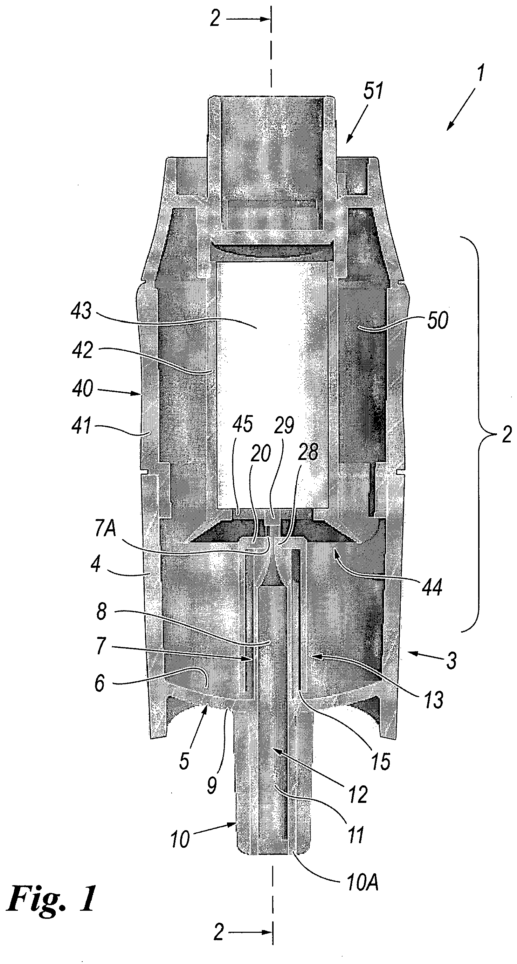

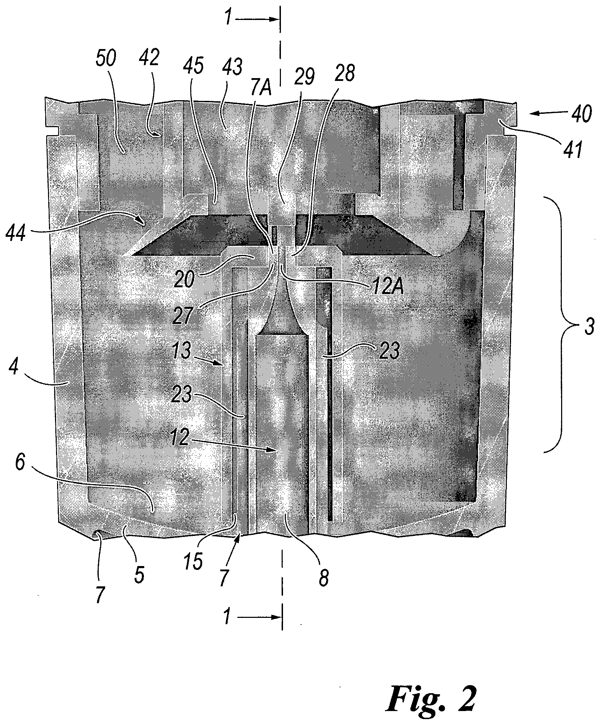

Figure 1 is a longitudinal section through a nebulizer according to the invention, said section being taken on the line 1-1 of Figure 2;
Figure 2 is a partial section on the line 2-2 of Figure 1;
Figure 3 is a partial perspective view, seen from above, of a portion of the nebulizer of Figure 1;
Figure 4 is an enlarged view of the portion shown in Figure 3;

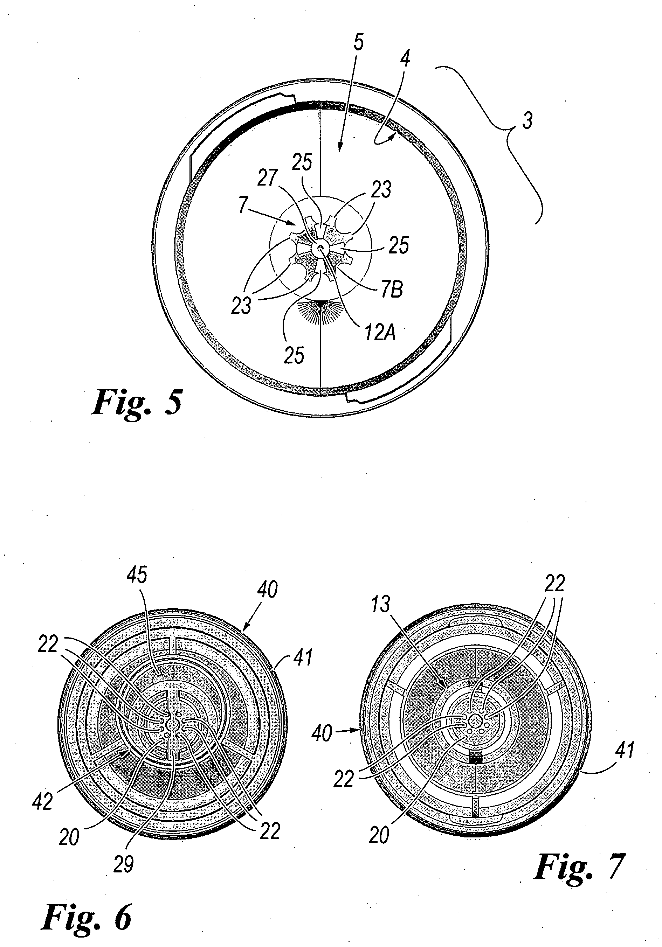

Figure 5 is a view of the portion of Figure 4, seen from above;
Figure 6 is a perspective view of a different part of the invention, seen from above;
Figure 7 is a view from below of that part shown in Figure 6;

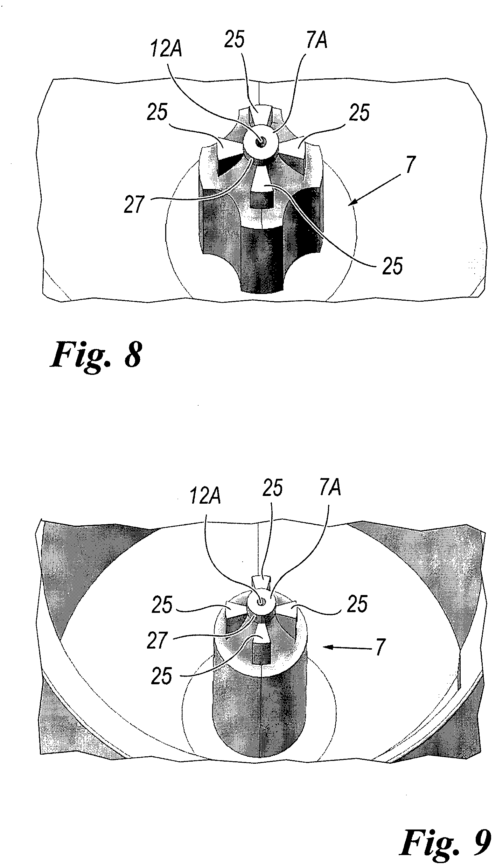

Figure 8 is a view similar to that of Figure 4, but of a variant of the nebulizer according to the invention;
Figure 9 is a view similar to that of Figure 4, but of a further variant of the nebulizer according to the invention; and

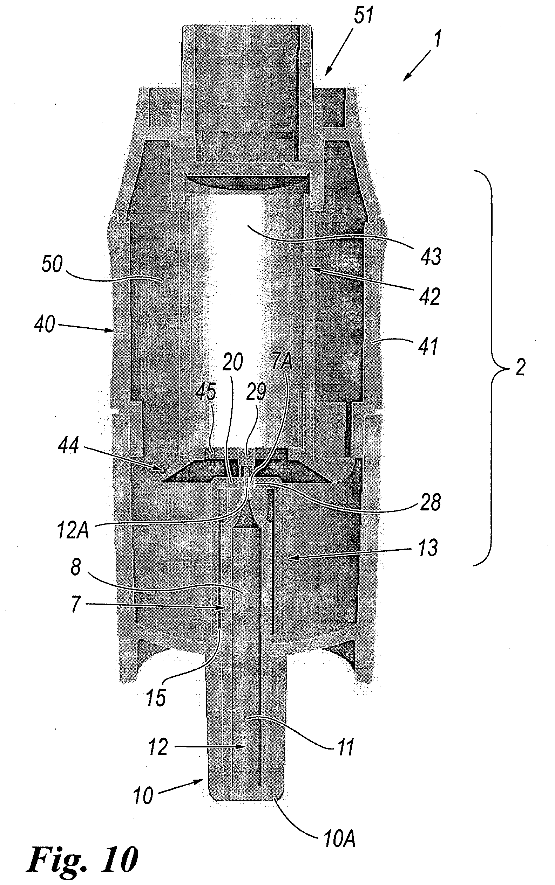

Figure 10 is a view similar to that of Figure 1, but of that variant of the nebulizer which comprises the part shown in Figure 9.

With reference to said figures, a nebulizer is indicated overall by 1 and comprises a body 2 presenting a cup-shaped first portion 3 arranged to act as a reservoir for a medical product, i.e. to contain the medical product to be dispensed. This portion or reservoir 3 presents a lateral part 4 and a base part 5; this latter comprises two opposing faces: a tubular body 7, hollow at 8, extends upwards from an internal face 6. A tubular body 10 hollow at 11 extends from the external face 9. The cavities 8 and 11 are connected together to form a single cavity 12 open at the free end 10A of the tubular body 10, to be connected to a source or generator of a forced flow of fluid (not shown), and open at the free end 7A of the tubular body 7 within the interior of the portion or reservoir 3.

A tube 13 is fitted onto the tubular body 7 coaxially to said body, but spaced laterally from it in order to form with this latter a space 15 enabling the product to rise from the reservoir 3. The product, driven by the venture effect by the fluid passing through the cavity 12 of the tubular body 7, rises towards the end 7A of this latter until it reaches a part 20 of the tube 13 disposed transversely above the end 7A. This transverse part comprises a plurality of apertures 22 enabling the fluid flowing along the tubular body 7 to emerge above this latter.

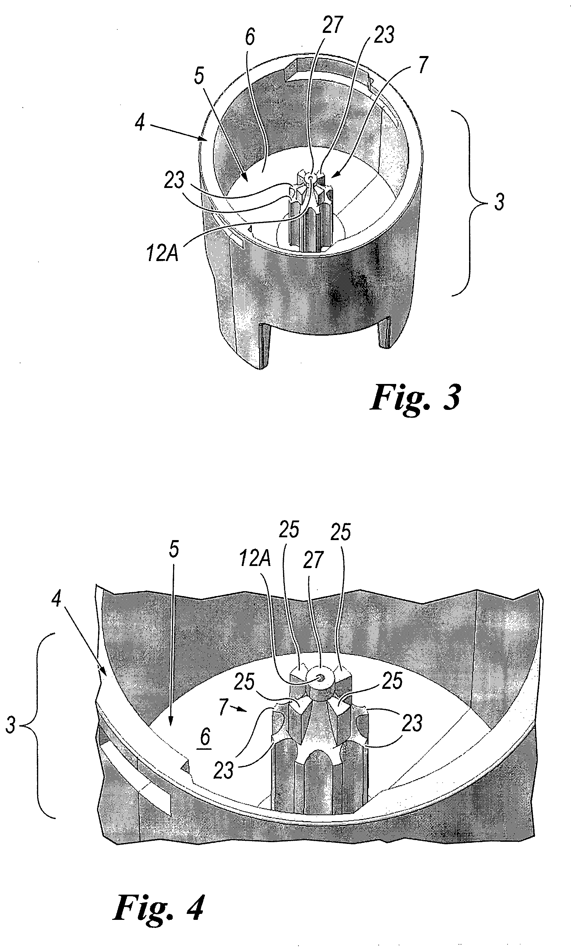

It should be noted that in a first embodiment (Figures 3 and 4) this tubular body comprises a plurality of fins 23 projecting longitudinally along that wall 7B thereof facing the tube 13, said fins defining, when the body 7 is covered by said tube, a series of capillary conduits 24 through which the product contained in the reservoir 3 moves. At its end 7A, the body 7 tapers and comprises a plurality of fins 25, positioned offset from the fins 23, on which the tube 13 rests. Between the fins 25 there is an end projection 27 (positioned centrally on the end 7A and having an opening 12A for the cavity 12) arranged to penetrate into a hole 28 of the tube 13 when this is fitted onto the body 7.

This tube comprises a bar 29 positioned at a distance from the transverse part 20, above the opening 12A of the cavity 12. The fluid emerging from this opening strikes against this bar after passing through the cavity 12. The apertures 22 are disposed about the hole 28 (essentially corresponding with the conduits 24) and enable the product to flow from the space 15 without it striking against the bar 29, the product also being drawn onto the surface of the tube part 20 by the fluid movement created by the impact against this bar.

The tube 13 is integral with a second portion 40 of the nebulizer body 2, said second portion comprising a perimetral wall 41 adapted to rest on the perimetral wall 4 of the first portion or reservoir 3, and a cylindrical central part 42 with a cavity 43, terminating with a perimetral flared portion 44 acting as a deflector above the tube 13. The cavity 43 is positioned above the bar 29 and is open via an opening 45 at which said bar is positioned. The edge of the opening is defined by the flared deflector portion 44, said portion or deflector being sufficiently large to be disposed about the part 20 of the tube 13. The deflector has the known function of causing the non-nebulized product droplets not extracted from the body 2 to fall back into the reservoir 3 through an annular conduit 50 positioned between the cylindrical part 42 and the perimetral wall 41 and connected to an opening (not shown) of a dispenser 51 superposed on the second portion 40 of the body 2.

The nebulizer of the invention therefore has a reduced number of components (the first portion 3, the second portion 40 and the dispenser 51) and is easily produced and assembled. In a variant, the dispenser of the nebulizer can be integral with the second portion 40. In addition to those described, other variants of the invention are possible in the light of the preceding description and are to be considered as falling within the scope of the following claims.

## Claims

1. A nebulizer comprising a body (2) presenting various portions coupled together, a first portion (3) of these portions defining a reservoir arranged to contain a medical product and being able to be connected to a generator of a forced flow of a fluid able to transport said product towards a dispenser (5), said first portion (3) comprising a hollow tubular body (7, 10) disposed in the reservoir, said hollow tubular body (7, 10) having a cavity (12) in communication with the flow generator, said tubular body (7) being coaxially surrounded by a tube (13) arranged such as to create with the tubular body (7) a space (15) through which the product is able to rise from the reservoir as far as the end (7A) of the tubular body (7), a part (20) of the tube (13) being positioned transversely above said tubular body (7) and above an end opening (12A) of the cavity (12), above said end opening (12A) there being positioned a deflector element (44) arranged to deviate at least part of the fluid and product flow towards a dispenser outlet, the tube (13) positioned above the tubular body (7) and the deflector element (44) forming a single piece, **characterised in that**:
- the deflector element (44) is part of a second portion (40) superposed on the first portion (3),
- said second portion (40) comprises a cylindrical central part (42) provided with a cavity (43) disposed and opening at an opening (45) above the tubular body (7) and above its superposed tube (13) of the first portion (3),
- said hollow cylindrical central part (42) terminates with a perimetral flared portion bounding the opening (45) of said central part (42) and defining the deflector element (44),
- the tube (13) superposed on the tubular body (7) of the first portion presents, in said part (20) of the tube (13) positioned transversely above said tubular body (7) and above said end opening (12A), a plurality of apertures (22) disposed circumferentially about a central opening (28) into which an end projection (27) of said tubular body (7) penetrates, in this end projection (27) there being provided the end opening (12A) of the cavity (12),
- said product emerging from said apertures (22).

2. A nebulizer as claimed in claim 1, **characterised in that** said second portion (40) presents a perimetral part (41) adapted to rest on a perimetral wall (4) of the first portion (3) and bounding the reservoir of this latter, between said perimetral wall (41) of said second portion and the cylindrical central part (42) of this latter there being provided an annular conduit (50) through which the nebulized product reaches the dispenser.

3. A nebulizer as claimed in claim 1, **characterised in that** the dispenser (51) is superposed on the second portion (40) of the nebulizer body (2) and is separate therefrom.

4. A nebulizer as claimed in claim 1, **characterised in that** the dispenser (51) is integral with the second portion (40) of the nebulizer body (2).

5. A nebulizer as claimed in claim 1, **characterised in that** said apertures (22) are arranged to correspond with capillary conduits (24) provided between the tube (13) and the tubular body (7) when superposed, and defined by longitudinal fins (23) projecting from that wall (7B) of said body (7) facing the tube (13).

6. A nebulizer as claimed in claim 1, **characterised in that** the end (7A) of the tubular body (7) comprises a plurality of fins (25) close to the end projection (27) and arranged preferably offset from the longitudinal fins (23), the tube (13) superposed on said tubular body (7) resting on this plurality of fins.

## Patentansprüche

1. Zerstäuber, umfassend einen Körper (2), der verschiedene, miteinander verbundene Abschnitte aufweist, wobei ein erster Abschnitt (3) dieser Abschnitte einen Sammelbehälter definiert, der angeordnet ist, um ein Medizinprodukt aufzunehmen, und in der Lage ist, mit einem Generator zur Erzeugung eines Zwangsdurchlaufs eines Fluides verbunden zu werden, der in der Lage ist, das Produkt zu einem Spender (5) zu fördern, wobei der erste Abschnitt (3) einen rohrförmigen Hohlkörper (7, 10) umfasst, der in dem Sammelbehälter angeordnet ist, wobei der rohrförmige Hohlkörper (7, 10) einen Hohlraum (12) aufweist, der mit dem Durchlaufgenerator in Verbindung steht, wobei der rohrförmige Körper (7) koaxial von einem Rohr (13) umgeben ist, das angeordnet ist, um mit dem rohrförmigen Körper (7) einen Raum (15) zu bilden, durch den das Produkt in der Lage ist, aus dem Sammelbehälter in Bezug auf das Ende (7A) des rohrförmigen Körpers (7) aufzusteigen, wobei ein Teil (20) des Rohrs (13) quer über dem rohrförmigen Körper (7) und über einer Endöffnung (12A) des Hohlraums (12) angeordnet ist, wobei über der Endöffnung (12A) ein Umlenkelement (44) angeordnet ist, um wenigstens einen Teil des Fluid- und Produktdurchlaufs in Richtung eines Spenderauslasses umzulenken, wobei das über dem rohrförmigen Körper (7) und dem Umlenkelement (44) angeordnete Rohr (13) ein einziges Teil bildet, **dadurch gekennzeichnet, dass**:
- das Umlenkelement (44) Teil eines zweiten Abschnitts (40) ist, der über dem ersten Abschnitt (3) liegt,
- der zweite Abschnitt (40) einen zylindrischen Mittelteil (42) umfasst, der mit einem Hohlraum (43) versehen ist, der angeordnet ist und sich öffnet an einer Öffnung (45) über dem rohrförmigen Körper (7) und über dessen überlappendem Rohr (13) des ersten Abschnitts (3),
- der hohle, zylindrische Mitteilteil (42) in einem konisch erweiterten, umlaufenden Abschnitt endet, der die Öffnung (45) des Mittelteils (42) begrenzt und das Umlenkelement (44) definiert,
- das über dem rohrförmigen Körper (7) des ersten Abschnitts liegende Rohr (13) in dem Teil (20) des Rohrs (13), der quer über dem rohrförmigen Körper (7) und über der Endöffnung (12A) angeordnet ist, eine Mehrzahl von Öffnungen (22) aufweist, die peripher um eine mittlere Öffnung (28) angeordnet sind, in die eine Endauskragung (27) des rohrförmigen Körpers (7) eindringt, wobei in der Endauskragung (27) die Endöffnung (12A) des Hohlraums (12) vorgesehen ist,
- wobei das Produkt aus den Öffnungen (22) austritt.

2. Zerstäuber nach Anspruch 1, **dadurch gekennzeichnet, dass** der zweite Abschnitt (40) einen umlaufenden Teil (41) aufweist, der dazu geeignet ist, auf einer umlaufenden Wand (4) des ersten Abschnitts (3) aufzuliegen und den Sammelbehälter des letzteren begrenzt, wobei zwischen der umlaufenden Wand (41) des zweiten Abschnitts und dem zylindrischen Mittelteil (42) des letzteren eine ringförmige Leitung (50) vorgesehen ist, durch die das zerstäubte Produkt den Spender erreicht.

3. Zerstäuber nach Anspruch 1, **dadurch gekennzeichnet, dass** der Spender (51) über dem zweiten Abschnitt (40) des Zerstäuberkörpers (2) liegt und von diesem getrennt ist.

4. Zerstäuber nach Anspruch 1, **dadurch gekennzeichnet, dass** der Spender (51) einstückig mit dem zweiten Abschnitt (40) des Zerstäuberkörpers (2) ausgeführt ist.

5. Zerstäuber nach Anspruch 1, **dadurch gekennzeichnet, dass** die Öffnungen (22) angeordnet sind, um mit Kapillarleitungen (24) zu korrespondieren, die zwischen dem Rohr (13) und dem rohrförmigen Körper (7) vorgesehen sind, wenn dieser darüber liegt, und durch Längsrippen (23) definiert sind, die aus der Wand (7B) des dem Rohr (13) gegenüberliegenden Körpers (7) auskragen.

6. Zerstäuber nach Anspruch 1, **dadurch gekennzeichnet, dass** das Ende (7A) des rohrförmigen Körpers (7) eine Mehrzahl von Rippen (25) umfasst, die in der Nähe der Endauskragung (27) und vorzugsweise zu den Längsrippen (23) versetzt angeordnet sind, wobei das über dem rohrförmigen Körper (7) liegende Rohr (13) auf dieser Mehrzahl von Rippen aufliegt.

## Revendications

1. Nébuliseur comprenant un corps (2) présentant diverses portions couplées ensemble, une première portion (3) de ces portions définissant un réservoir agencé pour contenir un produit médical et étant adapté pour être connecté à un générateur d'un flux forcé d'un fluide adapté pour transporter ledit produit vers un distributeur (5), ladite première portion (3) comprenant un corps tubulaire creux (7, 10) disposé dans le réservoir, ledit corps tubulaire creux (7, 10) ayant une cavité (12) en communication avec le générateur de flux, ledit corps tubulaire (7) étant entouré de manière coaxiale par un tube (13) agencé de manière à créer avec le corps tubulaire (7) un espace (15) à travers lequel le produit est capable de monter à partir du réservoir jusqu'à l'extrémité (7A) du corps tubulaire (7), une partie (20) du tube (13) étant positionné transversalement au-dessus dudit corps tubulaire (7) et au-dessus d'une ouverture d'extrémité (12A) de la cavité (12), au-dessus de ladite ouverture d'extrémité (12A) étant positionné un élément déflecteur (44) agencé pour dévier au moins une partie du flux de fluide et de produit vers une sortie de distributeur, le tube (13) positionné au-dessus du corps tubulaire (7) et l'élément déflecteur (44) formant une seule pièce, **caractérisé en ce que** :
- l'élément déflecteur (44) fait partie d'une deuxième portion (40) superposée sur la première portion (3),
- ladite deuxième portion (40) comprend une partie centrale cylindrique (42) munie d'une cavité (43) disposée et débouchant au niveau d'une ouverture (45) au-dessus du corps tubulaire (7) et au-dessus de son tube superposé (13) de la première portion (3),
- ladite partie centrale cylindrique creuse (42) se termine avec une portion évasée périphérique délimitant l'ouverture (45) de ladite partie centrale (42) et définissant l'élément déflecteur (44),
- le tube (13) superposé sur le corps tubulaire (7) de la première portion présente, dans ladite partie (20) du tube (13) positionnée transversalement au-dessus dudit corps tubulaire (7) et au-dessus de ladite ouverture d'extrémité (12A), une pluralité d'orifices (22) disposés de manière circonférentielle autour d'une ouverture centrale (28) dans laquelle pénètre une saillie terminale (27) dudit corps tubulaire (7), dans cette saillie terminale (27) étant disposée l'ouverture d'extrémité (12A) de la cavité (12),
- ledit produit sortant desdits orifices (22).

2. Nébuliseur selon la revendication 1, **caractérisé en ce que** ladite deuxième portion (40) présente une partie périphérique (41) adaptée pour reposer sur une paroi périphérique (4) de la première portion (3) et délimitant le réservoir de cette dernière, entre ladite paroi périphérique (41) de ladite deuxième portion et la partie centrale cylindrique (42) de celle-ci étant disposé un conduit annulaire (50) à travers lequel le produit nébulisé atteint le distributeur.

3. Nébuliseur selon la revendication 1, **caractérisé en ce que** le distributeur (51) est superposé sur la deuxième portion (40) du corps de nébuliseur (2) et est séparé de celle-ci.

4. Nébuliseur selon la revendication 1, **caractérisé en ce que** le distributeur (51) est solidaire de la deuxième portion (40) du corps de nébuliseur (2).

5. Nébuliseur selon la revendication 1, **caractérisé en ce que** lesdits orifices (22) sont agencés pour correspondre avec des conduits capillaires (24) disposés entre le tube (13) et le corps tubulaire (7) quand ils sont superposés, et définis par des ailettes longitudinales (23) faisant saillie à partir de cette paroi (7B) dudit corps (7) faisant face au tube (13).

6. Nébuliseur selon la revendication 1, **caractérisé en ce que** l'extrémité (7A) du corps tubulaire (7) comprend une pluralité d'ailettes (25) proches de la saillie terminale (27) et agencées de préférence décalées par rapport aux ailettes longitudinales (23), le tube (13) superposé sur ledit corps tubulaire (7) reposant sur cette pluralité d'ailettes.
